# EUROPÄISCHE PATENTSCHRIFT

(11) **EP 4 008 250 B1**
(45) Veröffentlichungstag und Bekanntmachung des Hinweises auf die Patenterteilung: **18.03.2026**
(21) Anmeldenummer: 21211105.8
(22) Anmeldetag: 29.11.2021
(51) Int. Cl.: A61B 5/08, A61M 16/00, A61B 5/0205, A61B 5/024, A61B 5/00, A61B 5/083, A61B 5/087, A61B 5/1455

(54) **BEATMUNGSGERÄT**
VENTILATOR APPARATUS
APPAREIL RESPIRATOIRE

(30) Priorität: 01.12.2020 DE 102020131836
(43) Veröffentlichungstag der Anmeldung: 08.06.2022
(73) Patentinhaber: Löwenstein Medical Technology S.A., 2557 Luxembourg (LU)
(72) Erfinder: Kremeier, Peter, 76149 Karlsruhe (DE); Marx, Thomas, 22525 Hamburg (DE)
(74) Vertreter: Löwenstein Medical IP

(56) Entgegenhaltungen:
- EP-A1- 3 254 617
- EP-B1- 2 343 097
- US-A- 5 704 345
- US-A1- 2010 036 266

## Beschreibung

Die vorliegende Erfindung betrifft ein Beatmungsgerät mit wenigstens einer Beatmungseinrichtung zu Erzeugung einer Atemgasströmung für eine Beatmung und mit wenigstens einer Überwachungseinrichtung zur Überwachung wenigstens einer charakteristischen Kenngröße der Atemgasströmung.

Mitunter können solche Beatmungsgeräte zur Beatmung während einer Herz-Lungen-Wiederbelebung (sog. kardiopulmonale Reanimation; englisch Cardiopulmonary Resuscitation, CPR) eingesetzt werden. Durch die geräteseitige Übernahme der Beatmung wird ein Arzt bzw. Helfer erheblich entlastet, sodass eine bessere Konzentration auf die Herzdruckmassage (HDM) und andere Maßnahmen möglich ist.

Im Stand der Technik sind Beatmungsgeräte bekannt geworden, welche bei einer CPR nicht nur die Beatmung bereitstellen, sondern auch bei der Ausführung der HDM unterstützen können. Dazu überwacht das Beatmungsgerät mittels Sensoren Druck- und Flussänderungen in der Lunge des Patienten während der HDM. Durch Auswertung der Sensorsignale erkennt das Gerät, wie oft und wie stark der Helfer einen Hub für die HDM ausführt. Beispielsweise kann das Gerät nach Erkennung von 30 Hüben dazu auffordern, die HDM zu unterbrechen, damit zwei Beatmungshübe durch das Gerät oder den Helfer erfolgen können. Eine Erkennung der (einsetzenden) Herzaktivität erfolgt hier über ein zusätzliches EKG.

Ein gattungsgemäßes Beatmungsgerät ist aus der EP 3 254 617 A1 und aus der US 2010/0036266 A1 bekannt.

Demgegenüber ist es die Aufgabe der vorliegenden Erfindung, die technischen Vorrichtungen zur Unterstützung einer CPR zu verbessern. Insbesondere soll eine Vorrichtung zur Verfügung gestellt werden, welche im Rahmen einer CPR eingesetzt werden kann und dabei zuverlässige und hilfreiche Informationen über den Patienten bzw. den Erfolg der CPR bereitstellt, insbesondere ohne ein zusätzliches EKG zu benötigen.

Diese Aufgabe wird gelöst mit einem Beatmungsgerät des Anspruchs 1. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die vorliegende Erfindung bietet viele Vorteile. Einen erheblichen Vorteil bietet der Erkennungsmodus zur Erkennung von Herzschlägen auf der Basis einer Auswertung der erfassten Kenngröße. Das ermöglicht eine technische unaufwendig umsetzbare und zugleich besonders zuverlässige und automatisierte Herschlagerkennung. So kann das Gerät bzw. System Herzschläge erkennen und optional dem Helfer mitteilen, ob und wann das Herz des Patienten wieder zu schlagen begonnen hat. Entsprechend ist der Helfer darüber informiert, ob die CPR bzw. HDM fortführen muss oder nicht.

Denn bei einer CPR ist es sehr entscheidend, die wieder einsetzende Herzaktivität möglichst früh zu erkennen, um entsprechend darauf reagieren zu können und weitere wichtige Maßnahmen einzuleiten. Bisher war die Erkennung der wieder einsetzenden Herzaktivität oft sehr schwierig, da die Konzentration des Helfers auf die CPR und insbesondere auf die HDM gerichtet ist. Mit der vorliegenden Erfindung kann sich der Helfer nun ganz auf die notwendigen Maßnahmen konzentrieren, ohne dass er ständig kontrollieren muss, ob das Herz wieder schlägt.

Dabei ist die technische bzw. konstruktive Umsetzung der Erfindung ein besonderer Vorteil, denn dadurch kann die Herzschlagerkennung wirtschaftlich und medizinisch aussagekräftig zur Verfügung gestellt werden kann. Da die Herzschläge durch die entsprechende Auswertung einer Kenngröße erkannt werden, welche ohnehin oft im Rahmen der Beatmung erfasst wird, ist beispielsweise eine Nachrüstung bereits bestehender Geräte besonders einfach möglich.

Erfindungsgemäß ist die wenigstens eine Kenngröße ein Maß für einen Fluss des Atemgases.

Insbesondere ist die Kenngröße der Atemgasfluss Es ist möglich, dass wenigstens zwei Kenngrößen registriert werden, umfassend wenigstens den Atemgasfluss und den Atemgasdruck. Im vorliegenden technischen Gebiet wird der (Atemgas-) Fluss häufig auch als Flow bezeichnet. Daher können die Begriffe (Atemgas-) Fluss und Flow im Rahmen der vorliegenden Erfindung synonym verwendet werden. Der (Atemgas-) Fluss ist insbesondere ein Volumenstrom. Das Atemgas ist im Sinne der Erfindung insbesondere solches Atemgas, welches dem Patienten von dem Beatmungsgerät zugeführt wird und/oder ein Atemgas, welches der Lunge des Patienten entweicht und/oder allgemein ein Atemgas, welches die Erkennung der erfindungsgemäßen Herzschlagsignale ermöglicht.

Bevorzugt ist das wenigstens eine Verlaufsstrukturmerkmal eine zeitliche Änderung der Kenngröße.

Insbesondere ist als Bedingung wenigstens ein Maß für eine Ähnlichkeit zu einer durch Herzschläge verursachten zeitlichen Änderung der Kenngröße vorgesehen. Insbesondere beschreibt das Verlaufsstrukturmerkmal, wie schnell und/oder stark und/oder häufig sich die Kenngröße über die Zeit ändert. Das Heranziehen solcher Kenngrößen bietet eine besonders zuverlässige und zugleich technisch besonders unaufwendig umsetzbare Erkennung von Herzschlägen.

Alternativ oder zusätzlich kann das wenigstens eine Verlaufsstrukturmerkmal eine geometrische Struktur des zeitlichen Verlaufs der Kenngröße beschreiben. Als Bedingung ist dann insbesondere wenigstens ein Maß für eine Ähnlichkeit zu einer hinterlegten und durch Herzschläge verursachten geometrischen Struktur des zeitlichen Verlaufs der Kenngröße vorgesehen. Solche Strukturen sind beispielsweise Symmetrieeigenschaften, Steigungen, Wendepunkte, Nullstellen, Hochpunkte, Tiefpunkte. Es können auch andere im Rahmen einer Funktionsanalyse bzw. Kurvendiskussion des zeitlichen Verlaufs der Kenngröße identifizierbare Merkmale herangezogen werden. Die Bedingung ist dann insbesondere eine Ähnlichkeit eines solchen Merkmals zu wenigstens einem hinterlegten Merkmal.

In einer bevorzugten und vorteilhaften Weiterbildung beschreibt das wenigstens eine Verlaufsstrukturmerkmal eine zeitliche Flussänderung oder ist eine solche. Insbesondere ist die Bedingung dann wenigstens, dass die zeitliche Flussänderung eine definierte Ähnlichkeit zu einer durch Herzschläge verursachten zeitlichen Flussänderung aufweist. Da spezifische Flussänderungen und Druckänderungen sehr verlässlich zusammen mit Herzschlägen auftreten, bietet eine solche Ausgestaltung eine besonders reproduzierbare Erkennung von Herzschlägen.

Vorzugsweise ist als Bedingung wenigstens vorgegeben, wie häufig und/oder regelmäßig das wenigstens eine Verlaufsstrukturmerkmal im zeitlichen Verlauf der Kenngröße auftritt. So können beispielsweise durch ein Rauschen bedingte Änderungen der Kenngröße gegenüber kardiogenen Änderungen diskriminiert werden.

Das wenigstens eine Verlaufsstrukturmerkmal beschreibt ein Auftreten von (lokalen) Maxima und/oder (lokalen) Minima im zeitlichen Verlauf der Kenngröße oder ist wenigstens ein solches. Es ist als Bedingung wenigstens eine maximale Grenze für einen Betrag der Kenngröße an einem Maximum oder Minimum vorgesehen. Eine solche Ausgestaltung ist besonders vorteilhaft, da bei Herzschlägen zwar signifikante Änderungen auftreten, die Maxima bzw. Minima aber in der Regel nur geringe Werte annehmen.

Insbesondere ist zusätzlich als Bedingung aber auch eine minimale Grenze für den Wert und/oder Betrag der Kenngröße an einem Maximum und/oder Minimum vorgesehen. Dadurch können unerhebliche Änderungen bzw. ein Rauschen ausgeblendet werden. Es ist möglich, dass dazu wenigstens ein Filter für die Daten vorgesehen ist.

Alternativ oder zusätzlich kann das wenigstens eine Verlaufsstrukturmerkmal wenigstens eines der folgenden Merkmale im zeitlichen Verlauf der Kenngröße beschreiben: Häufigkeit der Maxima und/oder Minima pro Zeiteinheit (Frequenz); Anzahl der Maxima und/oder Minima in einem Zeitraum; zeitliche Regelmäßigkeit des Auftretens; (geometrische) Verlaufsform der Maxima und/oder Minima. Insbesondere ist die Steuereinrichtung dazu geeignet und ausgebildet, die Herzaktivität wenigstens in Abhängigkeit davon zu erkennen, welche Häufigkeit und/oder welchen Wert und/oder welchen geometrischen Verlauf die Maxima und/oder Minima aufweisen.

Erfindungsgemäß ist, dass das wenigstens eine Verlaufsstrukturmerkmal einen maximalen und/oder minimalen Fluss beschreibt oder ein solcher ist. Als Bedingung ist erfindungsgemäß wenigstens eine Obergrenze für einen Betrag des Flusses vorgesehen. Dadurch können die spezifischen, aber entsprechend schwachen Flussänderungen zuverlässig erkannt werden.

Bei einem Maximum des Flusses liegt insbesondere ein positiver Fluss bzw. ein zum Patienten hinströmender bzw. in Richtung der Lunge des Patienten strömender Atemgasfluss vor. Bei einem Minimum des Flusses liegt insbesondere ein negativer Fluss bzw. ein entgegengesetzt strömender Atemgasfluss vor.

Erfindungsgemäß ist als Bedingung wenigstens eine Obergrenze für einen Betrag eines Flusses des Atemgases vorgesehen. Die Obergrenze liegt zwischen 0,01 l/s und 0,3 l/s und vorzugsweise zwischen 0,02 l/s und 0,15 l/s. Insbesondere als Bedingung wenigstens vorgesehen, dass das Maximum des Flusses nicht größer 0,3 l/s. Insbesondere als Bedingung wenigstens vorgesehen, dass das Minimum des Flusses nicht kleiner als - 0,02 l/s ist. Zusätzlich oder alternativ kann als Bedingung auch eine Obergrenze für den Betrag einer anderen Kenngröße und vorzugsweise des Druckes des Atemgases vorgesehen sein. So können die für Herzschläge spezifischen Flussänderungen besonders gut identifiziert werden.

Es ist möglich, dass als Bedingung wenigstens vorgesehen ist, dass zwischen einem Maximum und einem Minimum im zeitlichen Verlauf der Kenngröße wenigstens ein Vorzeichenwechsel erfolgt. Insbesondere erfolgt zwischen einem Maximum und einem Minimum im zeitlichen Verlauf des Flusses und/oder des Druckes des Atemgases wenigstens ein Vorzeichenwechsel und vorzugsweise nur ein Vorzeichenwechsel. Ein solcher Vorzeichenwechsel ist insbesondere durch das Kontrahieren und Entspannen des schlagenden Herzens und dessen Auswirkung auf die Lunge bzw. den Brustraum bedingt.

Möglich ist auch, dass als Bedingung wenigstens vorgesehen ist, dass zeitliche Änderungen der Kenngröße und vorzugsweise ein Auftreten von Maxima und/oder Minima im zeitlichen Verlauf der Kenngröße mit einer definierten Häufigkeit und/oder Regelmäßigkeit im zeitlichen Verlauf der Kenngröße erfolgen. Die Häufigkeit beschreibt insbesondere die Anzahl der Änderungen innerhalb einer definierten Zeitspanne und kann auch als Frequenz bezeichnet werden. Die Regelmäßigkeit beschreibt insbesondere die zeitlichen Abstände zwischen den Änderungen und vorzugsweise zwischen den Maxima bzw. Minima.

Als Bedingung kann auch wenigstens vorgesehen sein, dass zeitliche Änderungen der Kenngröße und vorzugsweise ein Auftreten von Maxima und/oder Minima im zeitlichen Verlauf der Kenngröße mit einer Frequenz von wenigstens zehn pro Minute und vorzugsweise 20 pro Minute und insbesondere im Bereich 30 bis 200 pro Minute erfolgen. Insbesondere entspricht die Frequenz wenigstens einer sehr niedrigen Herzfrequenz. Insbesondere beträgt die Frequenz, beispielsweise aufgrund der verabreichten Reanimationsmedikamente, maximal 180 pro Minute. Insbesondere entspricht die maximale Frequenz einer hohen Herzfrequenz. Es kann vorgesehen sein, dass die Steuereinrichtung die Frequenz dadurch festlegt, dass ein Lebensalter des Patienten abgefragt wird. So kann für jüngere Menschen und insbesondere Babys eine höhere (maximale und/oder minimale) Frequenz als für ältere Menschen eingestellt werden.

In einer vorteilhaften Weiterbildung ist die Steuereinrichtung dazu geeignet und ausgebildet, den zeitlichen Verlauf der Kenngröße auf wenigstens ein hinterlegtes herzschlagbedingtes Muster zu untersuchen. Insbesondere ist die Steuereinrichtung dazu geeignet und ausgebildet, Herzschläge wenigstens dadurch zu erkennen, dass das Muster wenigstens näherungsweise im zeitlichen Verlauf auftritt. Insbesondere ist das Muster durch Wiederholungen wenigstens eines Verlaufsstrukturmerkmals und vorzugsweise von wenigstens zwei Verlaufsstrukturmerkmalen definiert. Besonders bevorzugt beschreibt das Muster sich wiederholende geometrische Strukturen des zeitlichen Verlaufs der Kenngröße. Besonders bevorzugt definiert das Muster eine zeitliche und/oder geometrische Regelmäßigkeit von Flussänderungen und/oder Druckänderungen im zeitlichen Verlauf der Kenngröße. Die Bedingung ist dann insbesondere eine Ähnlichkeit des zeitlichen Verlaufs der Kenngröße zu wenigstens einem hinterlegten Muster.

Es ist möglich und vorteilhaft, dass die Steuereinrichtung dazu geeignet und ausgebildet ist, eine Häufigkeit des wenigstens einen Verlaufsstrukturmerkmals im zeitlichen Verlauf der Kenngröße zu bestimmen und aus der Häufigkeit eine Anzahl von Herzschlägen und/oder eine Herzfrequenz zu bestimmen. Die Anzahl von Herzschlägen und/oder die Herzfrequenz können auch aus dem Muster ermittelt werden. So kann die Herzaktivität besser eingeschätzt werden, nachdem sie wieder eingesetzt hat.

In einer bevorzugten und vorteilhaften Weiterbildung ist die Steuereinrichtung dazu geeignet und ausgebildet, die Erkennung der Herzschläge (auch) unter Berücksichtigung davon vorzunehmen, ob das wenigstens eine Verlaufsstrukturmerkmal mit einer Mindestqualität und/oder Mindestanzahl im registrierten zeitlichen Verlauf der Kenngröße aufgetreten ist. Die Mindestanzahl betrifft insbesondere die Anzahl der Maxima und/oder Minima. Möglich ist auch, die Erkennung der Herzschläge unter Berücksichtigung davon vorzunehmen, ob der zeitliche Verlauf der Kenngröße der Atemgasströmung während einer Mindestdauer registriert wurde und/oder ob der zeitliche Verlauf der Kenngröße der Atemgasströmung mit einer definierten Mindestsignalqualität registriert wurde. Die Mindestqualität definiert insbesondere, ob das Verlaufsstrukturmerkmal als solches erkennbar und auswertbar ist. Insbesondere ist die Steuereinrichtung dazu geeignet und ausgebildet, in Abhängigkeit der zuvor genannten Anforderungen das Ergebnis der Erkennung der Herzschläge zu verwerfen und/oder zu gewichten bzw. zu verifizieren.

Insbesondere beträgt die Mindestdauer wenigstens 2 oder 3 Sekunden und vorzugsweise wenigstens 5 Sekunden und besonders bevorzugt wenigstens 10 Sekunden.

In einer besonders vorteilhaften Ausgestaltung ist mittels der Überwachungseinrichtung ein Kohlendioxidgehalt des Blutes und/oder des Atemgases und/oder ein Blutdruck und/oder eine Sauerstoffkonzentration des Blutes als wenigstens eine weitere Kenngröße erfassbar. Vorzugsweise ist die Steuereinrichtung dazu geeignet und ausgebildet, einen zeitlichen Verlauf der wenigstens einen weiteren Kenngröße zu registrieren und für die Erkennung der Herzschläge wenigstens teilweise zu berücksichtigen. Bevorzugt ist die Steuereinrichtung dazu geeignet und ausgebildet, unter Berücksichtigung der weiteren Kenngröße eine Plausibilitätsüberprüfung durchzuführen, um das Ergebnis der Erkennung der Herzaktivität bzw. die erkannten Herzschläge zu verifizieren. Die Untersuchung der wenigstens einen weiteren Kenngröße erfolgt vorzugsweise so, wie es zuvor für die Kenngröße beschrieben wurde. Insbesondere erfolgt für die Erkennung von Herzschlägen eine gewichtete Berücksichtigung der Kenngröße und der weiteren Kenngröße. Der Kohlendioxidgehalt des Blutes wird dabei insbesondere durch einen Kohlendioxid-Partialdruck beschrieben. Insbesondere weist die Überwachungseinrichtung dazu geeignete Sensormittel auf und/oder ist mit wenigstens einem externen Sensormittel wirkverbunden. Bevorzugt weist die Überwachungseinrichtung wenigstens ein Sensormittel zur Erfassung des Kohlendioxidgehalt des Atemgases auf. Vorzugsweise wird die einsetzende Herztätigkeit durch einen steigenden Kohlendioxidgehalt des Blutes erkannt. Der Kohlendioxidgehalt des Blutes würde im weiteren Verlauf wieder absinken.

In allen Ausgestaltungen ist es besonders bevorzugt, dass die weitere Kenngröße eine insbesondere mittels Pulsoxymetrie erfasste Plethysmogramm-Welle und/oder eine mittels Pulsoxymetrie erfasste bzw. gemessene Sauerstoffkonzentration ist. Insbesondere ist die Überwachungseinrichtung dazu geeignet und ausgebildet, die weitere Kenngröße mittels einer Pulsoxymetrie bzw. pulsoxymetrisch zu erfassen. Dazu kann die Überwachungseinrichtung mit wenigstens einem Pulsoxymeter wirkverbunden sein oder selbst ein Pulsoxymeter aufweisen.

In einer besonders vorteilhaften Ausgestaltung ist mittels der Überwachungseinrichtung ein mittels Pulsoxymetrie gemessenen Plethysmogrammwelle und/oder mittels Pulsoxymetrie gemessenen Sauerstoffsättigung als wenigstens eine weitere Kenngröße erfassbar. Vorzugsweise ist die Steuereinrichtung dazu geeignet und ausgebildet, einen zeitlichen Verlauf der wenigstens einen weiteren Kenngröße zu registrieren und für die Erkennung der Herzschläge wenigstens teilweise zu berücksichtigen. Die Untersuchung der wenigstens einen weiteren Kenngröße erfolgt vorzugsweise so, wie es zuvor für die Kenngröße beschrieben wurde. Insbesondere erfolgt für die Erkennung von Herzschlägen eine gewichtete Berücksichtigung der Kenngröße und der weiteren Kenngröße. Insbesondere weist die Überwachungseinrichtung dazu geeignete Sensormittel auf und/oder ist mit wenigstens einem externen Sensormittel wirkverbunden. Insbesondere wird die einsetzende Herztätigkeit durch eine steigende Sauerstoffsättigung erkannt und/oder durch eine wiederkehrende Plethysmogrammwelle.

Es kann vorgesehen sein, dass die Steuereinrichtung dazu geeignet und ausgebildet ist, zur Überwachung einer Herzdruckmassage einen zeitlichen Verlauf der Kenngröße und vorzugsweise eines Flusses und/oder Drucks des Atemgases wenigstens während der Herzdruckmassage zu registrieren und eine Wirkung der Herzdruckmassage in Abhängigkeit davon zu bewerten, dass der zeitliche Verlauf eine definierte zeitliche Flussänderung und/oder Druckänderung aufweist. Insbesondere wird dazu die Qualität (insbesondere der ausgeübte Druck und/oder der Weg des Hubs) und/oder die Quantität (insbesondere die Anzahl der Hübe) bewertet.

Die Qualität wird vorzugsweise anhand der Amplitude der sensorisch durch die Steuereinheit erfassten Drücke und/oder Flüsse ermittelt. Die Quantität wird vorzugsweise anhand der Frequenz der sensorisch durch die Steuereinheit erfassten Drücke und/oder Flüsse ermittelt werden.

In allen Ausgestaltungen ist es besonders bevorzugt, dass die Steuereinrichtung dazu geeignet und ausgebildet ist, in dem Erkennungsmodus bzw. für eine Erkennung der Herzschläge wenigstens einen Hinweis auszugeben, dass eine Herzdruckmassage ausgesetzt und/oder wieder begonnen werden soll. Vorzugsweise wird zu Beginn des Erkennungsmodus bzw. vor der Durchführung der Erkennung der Herzschläge ein Hinweis ausgegeben, dass die Herzdruckmassage ausgesetzt werden soll. Falls die Herzdruckmassage fortgesetzt wird, erfolgt vorzugsweise wenigstens ein weiterer Hinweis und/oder das Ergebnis der Erkennung wird verworfen. Insbesondere wird am Ende des Erkennungsmodus bzw. nach einer Durchführung der Erkennung der Herzschläge ein Hinweis ausgegeben, dass die Herzdruckmassage wieder gestartet werden soll. Insbesondere wird das Aussetzen und/oder Fortsetzen der Herzdruckmassage mittels der Überwachungseinrichtung überwacht.

Insbesondere wird ein akustischer und/oder optischer und/oder haptischer Hinweis ausgegeben. Beispielsweise kann ein Signalton mit einem Texthinweis erfolgen. Bevorzugt ist auch, dass wenigstens eine Sprachausgabe erfolgt, welche aussagt, dass die Herzdruckmassage ausgesetzt bzw. wieder begonnen werden soll.

Möglich und vorteilhaft ist auch, dass im Erkennungsmodus bzw. für eine Erkennung der Herzschläge wenigstens ein Hinweis ausgegeben wird, dass eine Beatmung ausgesetzt und/oder wieder begonnen werden soll. Die Steuereinrichtung kann dazu geeignet und ausgebildet sein, die Beatmung im Erkennungsmodus wenigstens zeitweise zu unterbrechen. Möglich ist auch, dass die Beatmung im Erkennungsmodus wenigstens zeitweise fortgesetzt wird. Das Aussetzen kann in Abhängigkeit der Signalqualität erfolgen. Beispielsweise wird die Beatmung dann unterbrochen, wenn besonders schwache Druck- und/oder Flussänderungen erfasst werden müssen oder die Druck- und/oder Flusssignale nicht eindeutig und z. B. zu klein oder verrauscht sind.

Vorzugsweise kann die Herzschlagerkennung auch während der Beatmung oder während der HDM erfolgen. Die Steuereinrichtung erkennt dann insbesondere auch die die Druck- und/oder FlussSignale im Atemgas, die durch die (einsetzende) Herztätigkeit generiert werden.

Vorzugsweise ist die Steuereinrichtung dazu geeignet und ausgebildet, wenigstens einen akustischen und/oder optischen und/oder haptischen Unterstützungshinweis für eine Herzdruckmassage und vorzugsweise für deren Rhythmus vorzugeben.

Insbesondere ist die Steuereinrichtung dazu geeignet und ausgebildet, in Abhängigkeit eines Ergebnisses der Erkennung der Herzschläge wenigstens einen Hinweis auszugeben und vorzugsweise ein Vorliegen von Herzschlägen akustisch und/oder optisch und/oder haptisch zu signalisieren. So kann auch unter schwierigen Bedingungen wahrgenommen werden, dass das Herz wieder schlägt.

Besonders bevorzugt ist die Beatmungseinrichtung mittels der Steuereinrichtung in wenigstens einem Betriebsmodus zur Verwendung des Beatmungsgerätes in Kombination mit einer (separat ausgeführten) Herzdruckmassage betreibbar. Dabei stellt die Beatmungseinrichtung in einem solchen Betriebsmodus (hier als CPR-Betriebsmodus bezeichnet) wenigstens eine für eine kardiopulmonale Reanimation (CPR) spezifische Beatmung bereit. Eine solche Ausgestaltung ist besonders vorteilhaft, da zusätzlich zu der Überwachung der Herzaktivität noch eine weitere Unterstützung des Helfers dadurch erfolgt, dass auch die Beatmung vom Beatmungsgerät übernommen wird. Für eine solche Beatmung wird die Beatmungseinrichtung vorzugsweise unter Berücksichtigung der Kenngröße von der Steuereinrichtung angesteuert. Die Erfassung und Auswertung der Kenngröße erfolgt dabei vorzugsweise wie zuvor beschrieben. Insbesondere erfolgt die Beatmung in Abhängigkeit der Herzdruckmassage. Beispielsweise erfolgen nach 30 Hüben der Herzdruckmassage zwei Beatmungshübe durch die Beatmungseinrichtung.

Die Steuereinrichtung ist vorzugsweise dazu geeignet und ausgebildet, die Beatmung des CPR-Betriebsmodus während des Erkennungsmodus (gezielt) auszusetzen und vorzugsweise die Beatmung des CPR-Betriebsmodus nach dem Erkennungsmodus in Abhängigkeit eines Ergebnisses der Erkennung wieder (gezielt) fortzusetzen. Insbesondere ist die Steuereinrichtung dazu geeignet und ausgebildet, einen automatischen und/oder adaptiven Wechsel zwischen Erkennungsmodus und CPR-Betriebsmodus vorzunehmen. Insbesondere wird das Ergebnis der Erkennung der Herzschläge verworfen, wenn eine Beatmung und/oder Atemtätigkeit während der Erkennung vorliegt. Möglich ist aber auch, dass die Beatmung des CPR-Betriebsmodus während des Erkennungsmodus wenigstens teilweise fortgesetzt wird. Dazu kann beispielsweise ein angepasster Druck und/oder Fluss für die Beatmungseinrichtung vorgesehen sein. Möglich ist auch, dass der CPR-Betriebsmodus und der Erkennungsmodus wenigstens zeitweise parallel aktiv sind. Mit dem Aussetzen der Beatmung wird vorzugsweise auch der zuvor beschriebene Hinweis ausgegeben, dass die Herzdruckmassage ausgesetzt werden soll.

Die Überwachungseinrichtung ist vorzugsweise dazu geeignet und ausgebildet, die Kenngröße auch während des CPR-Betriebsmodus und insbesondere während der Durchführung einer HDM zu erfassen. Die Steuereinrichtung ist vorzugsweise dazu geeignet und ausgebildet, die Kenngröße auch während des CPR-Betriebsmodus und insbesondere während der Durchführung einer HDM registrieren und zu untersuchen.

Vorzugsweise ist als Bedingung wenigstens vorgesehen ist, dass eine zeitliche Änderung der Kenngröße, vorzugsweise Flussänderung und/oder Druckänderung, wenigstens eine Amplitude aufweist, deren Größe maximal die Hälfte, vorzugsweise maximal ein Viertel, der Größe wenigstens einer Amplitude beträgt, welche die zeitliche Änderung der Kenngröße während der Durchführung einer HDM aufweist. Insbesondere ist die Amplitude während der Durchführung einer HDM wenigstens doppelt so groß wie die Amplitude bei einem Herzschlag. Insbesondere ist als Bedingung wenigstens vorgesehen ist, dass solche Amplitude innerhalb eines definierten Zeitraums und/oder mit einer definierten Häufigkeit und/oder Regelmäßigkeit auftritt. In einer solchen Ausgestaltung sind insbesondere zeitliche Änderungen der Kenngröße und z. B. Amplitude gemeint, welche durch die Ausübung der CPR bedingt sind, also durch einen extern ausgeübten Druck auf den Brustkorb bzw. das Herz verursacht sind.

Bevorzugt ist als Bedingung wenigstens vorgesehen ist, dass eine zeitliche Änderung der Kenngröße, vorzugsweise Flussänderung und/oder Druckänderung, wenigstens eine Amplitude aufweist, deren Größe maximal die Hälfte, vorzugsweise maximal ein Drittel, der Größe wenigstens einer Amplitude beträgt, welche für den Druck und/oder Fluss des Atemgases für die Beatmung während des CPR-Betriebsmodus vorgesehen bzw. eingestellt ist. Insbesondere ist die Amplitude während der Beatmung im Rahmen einer CPR wenigstens drei Mal so groß wie die Amplitude bei einem Herzschlag. Insbesondere ist als Bedingung wenigstens vorgesehen ist, dass solche Amplitude innerhalb eines definierten Zeitraums und/oder mit einer definierten Häufigkeit und/oder Regelmäßigkeit auftritt.

Insbesondere ist die Steuereinrichtung dazu geeignet und ausgebildet ist, zur Überwachung einer Herzdruckmassage einen zeitlichen Verlauf eines Flusses und/oder Drucks des Atemgases wenigstens während der Herzdruckmassage (HDM) zu registrieren und eine Wirkung der Herzdruckmassage in Abhängigkeit davon zu bewerten, dass der zeitliche Verlauf eine definierte zeitliche HDM-Flussänderung und/oder Druckänderung aufweist, wobei zudem Flussänderungen und/oder Druckänderungen erfassbar sind, welche durch ein schlagendes Herz verursacht sind und wobei die Steuereinrichtung dazu geeignet und ausgebildet ist, die Herzschlag-Flussänderungen und/oder Druckänderungen in einem registrierten zeitlichen Verlauf des Flusses und/oder Drucks als Herzschläge zu erkennen. Vorzugsweise ist die Steuereinrichtung dazu geeignet und ausgebildet, die HDM-Flussänderung und/oder Druckänderung von der Herzschlag-Flussänderung und/oder Druckänderung dadurch zu unterscheiden, dass die HDM-Flussänderung und/oder Druckänderung eine zumindest doppelt so große Amplitude aufweisen wie die Herzschlag-Flussänderung und/oder Druckänderung.

Insbesondere ist die Steuereinrichtung dazu geeignet und ausgebildet, zur Überwachung der Beatmung einen zeitlichen Verlauf eines Flusses und/oder Drucks des Atemgases wenigstens während der Beatmung zu registrieren wobei zudem Flussänderungen und/oder Druckänderungen erfassbar sind, welche durch ein schlagendes Herz verursacht sind und wobei die Steuereinrichtung dazu geeignet und ausgebildet ist, die Herzschlag-Flussänderungen und/oder Druckänderungen in einem registrierten zeitlichen Verlauf des Flusses und/oder Drucks als Herzschläge zu erkennen. Vorzugsweise ist die Steuereinrichtung dazu geeignet und ausgebildet ist, die Beatmungs-Flussänderung und/oder Druckänderung von der Herzschlag-Flussänderung und/oder Druckänderung dadurch zu unterscheiden, dass die Beatmungs-Flussänderung und/oder Druckänderung eine zumindest dreifach so große Amplitude aufweisen wie die Herzschlag-Flussänderung und/oder Druckänderung.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Überwachungssystems in einer perspektivischen Ansicht;
- Fig. 2: ein stark schematisiertes Schaubild eines zeitlichen Verlaufs einer Kenngröße zur Erläuterung der Erkennung von Herzschlägen mittels der Erfindung; und
- Fig. 3: ein stark schematisiertes Schaubild eines zeitlichen Verlaufs einer weiteren Kenngröße zur Erläuterung der Erkennung von Herzschlägen mittels der Erfindung.

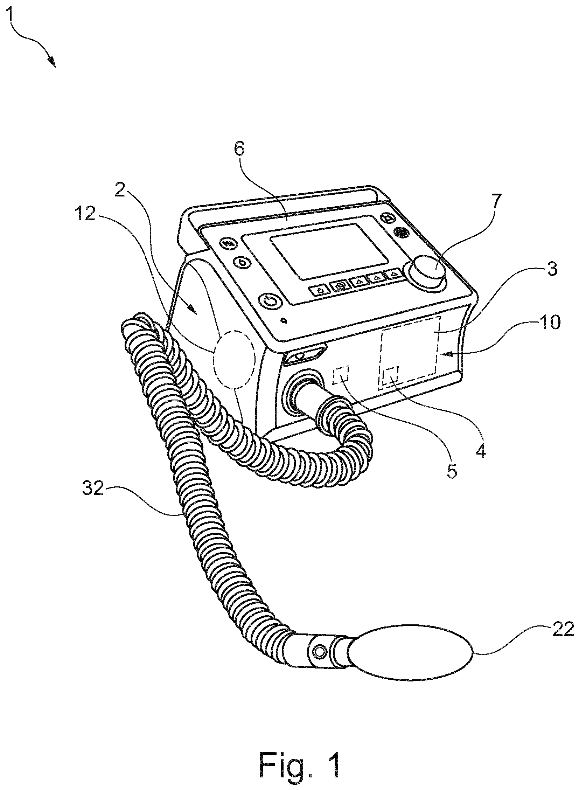

Die Figur 1 zeigt ein erfindungsgemäßes Beatmungsgerät 1, welches mit einem Überwachungssystem 10 mit einer Überwachungseinrichtung 3 ausgestattet ist. Das Überwachungssystem 10 kann alternativ auch als separate Vorrichtung außerhalb des Beatmungsgeräts 1 verwendet werden.

Das Beatmungsgerät 1 weist im Inneren seines Gehäuses hier eine Beatmungseinrichtung 2 auf, welche mit einer Lüftereinrichtung 12 zur Erzeugung einer Atemgasströmung ausgestattet ist. Die Atemgasströmung wird über eine an die Beatmungseinrichtung 2 gekoppelte Schlaucheinrichtung 32 mit einer Atemmaske 22 dem Patienten zugeführt. Alternativ zur Atemmaske 22 können auch andere Patientenschnittstellen verwendet werden. Zusätzlich oder alternativ zur Lüftereinrichtung 12 kann auch eine Druckgasquelle vorgesehen sein.

Die Überwachungseinrichtung 3 dient zur Überwachung charakteristischer Kenngrößen der Atemgasströmung. Die Beatmungseinrichtung 2 wird dann unter Berücksichtigung der Kenngröße und ggf. weiterer Beatmungsparameter mittels einer Steuereinrichtung 4 angesteuert.

Das Beatmungsgerät 1 umfasst hier eine Ausgabeeinrichtung 6 mit einer Anzeige bzw. einem Display und eine Bedieneinrichtung 7. Dabei können auch Kombinationen von Bedieneinrichtung 7 und Ausgabeeinrichtung 6 vorgesehen sein, beispielsweise in der Art einer berührungsempfindlichen Anzeigefläche bzw. eines Touchscreens. Die Ausgabeeinrichtung 6 dient hier auch zur Ausgabe von Hinweisen bzw. Signalen im Rahmen eines später beschriebenen Erkennungsmodus bzw. CPR-Betriebsmodus. Die Ausgabeeinrichtung 6 kann ihre Informationen bzw. Hinweise aber auch auf weiteren hier nicht gezeigten Anzeigeeinrichtungen ausgeben, z. B. auf einem Computerdisplay oder einem Tablet oder Smartphone oder dergleichen.

Die Beatmungseinrichtung 2 ist hier mit einem Sensormittel 5 wirkverbunden, welches mehrere Sensoren zur Erfassung der charakteristischen Kenngrößen der Atemgasströmung und ggf. weiterer für die Beatmung charakteristischer Größen aufweist.

Zum Beispiel umfasst das Sensormittel 5 einen hier nicht näher gezeigten Drucksensor, welcher die Druckverhältnisse der Atemgasströmung erfasst, sowie einen ebenfalls nicht näher gezeigten Flusssensor, welcher die Flussverhältnisse der Atemgasströmung erfasst.

Das Sensormittel 5 ist mit der Überwachungseinrichtung 3 und der Steuereinrichtung 4 wirkverbunden, sodass die erfassten Größen wenigstens teilweise von diesen verarbeitet werden können. Das Sensormittel 5 kann im Beatmungsgerät 1 oder im Schlauch oder in der Patientenschnittstelle angeordnet sein.

Mit der hier nicht sichtbar innerhalb des Gehäuses angeordneten Steuereinrichtung 4 wird die Lüftereinrichtung 12 angesteuert, sodass z. B. eine CPAP oder eine APAP- oder Bilevel-Beatmung ausgeführt werden können. Für eine Beatmung wird die Beatmungseinrichtung 2 z. B. auf einen definierten Atemgasfluss und/oder ein Atemgasdruck eingestellt. Die Steuereinrichtung 4 kann einen notwendigen Minimaldruck bereitstellen und/oder Druckschwankungen kompensieren, die durch die Atemtätigkeit des Benutzers bedingt sind. Beispielsweise erfasst die Überwachungseinrichtung 3 mittels des Sensormittels 5 den gegenwärtigen Druck in der Patientenschnittstelle 22 und regelt die Leistung der Lüftereinrichtung 12 entsprechend nach, bis ein gewünschter Beatmungsdruck anliegt.

Das Beatmungsgerät 1 kann in einem Erkennungsmodus für eine Herzaktivität betrieben werden. Im Erkennungsmodus erfolgt hier eine automatische Herzschlagerkennung. So kann das Beatmungsgerät 1 z. B. im Rahmen einer CPR feststellen, ob und wann das Herz wieder zu schlagen beginnt.

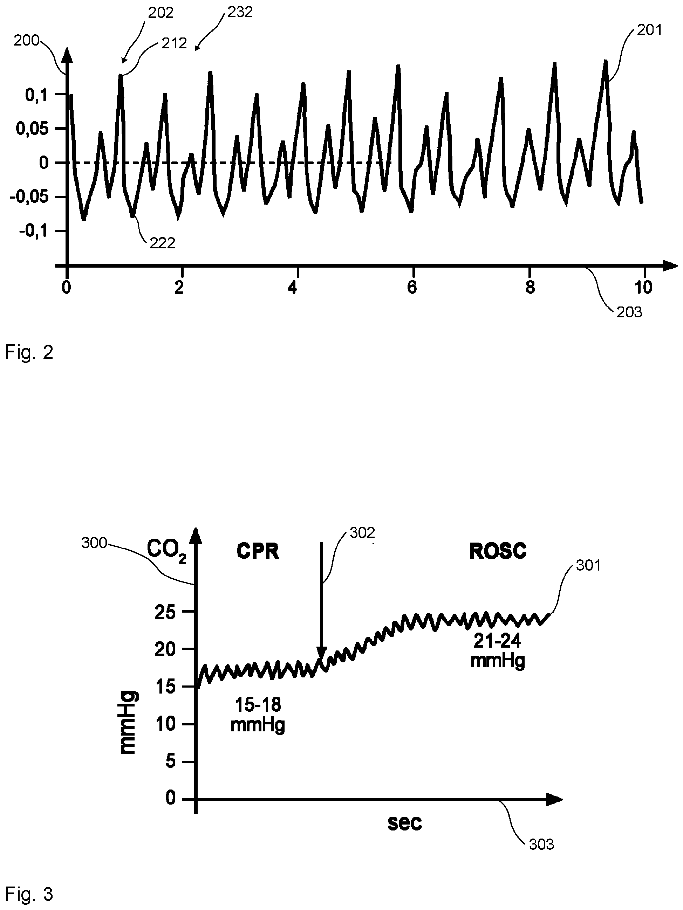

Ein beispielhafter Ablauf des Erkennungsmodus wird nun mit Bezug zu der Figur 2 beschrieben. Im Erkennungsmodus wertet die Steuereinrichtung 4 einen zeitlichen Verlauf 201 der Kenngröße 200 der Atemgasströmung aus, welchen die Überwachungseinrichtung 3 zuvor (als Druck oder Fluss oder Volumen) sensorisch erfasst hat und welcher von der Steuereinrichtung 4 registriert wurde. Der Verlauf 201 entspricht hier einer Auftragung der Kenngröße 200 über die Zeit 203, welche in Sekunden angegeben ist. Der hier gezeigte Verlauf 201 zeigt ein 10-sekündiges Fenster einer Aufzeichnung des Flusses.

Die Steuereinrichtung 4 untersucht dann den zeitlichen Verlauf 201 auf ein oder mehrere Verlaufsstrukturmerkmale 202. Wenn die untersuchten Verlaufsstrukturmerkmale 202 dann wenigstens eine in der Steuereinrichtung 4 hinterlegte Bedingung erfüllen und beispielsweise hinterlegten herzschlagspezifischen Verlaufsstrukturmerkmalen 202 entsprechen bzw. ähneln, wertet die Steuereinrichtung 4 dies als Vorliegen eines Herzschlages.

In dem hier gezeigten Beispiel entspricht die Kenngröße einem Fluss der Atemgasströmung. Zur Erkennung von Herzschlägen wird hier somit ein zeitlicher Verlauf 201 des Flusses herangezogen. Der Fluss ist hier in Liter pro Sekunde angegeben. Für die Auswertung untersucht die Steuereinrichtung 4 den Verlauf 201 auf spezifische Flussänderungen als Verlaufsstrukturmerkmal 202, welche durch das schlagende Herz verursacht sind. Alternativ oder zusätzlich kann als Kenngröße auch ein Druck der Atemgasströmung erfasst und in entsprechender Weise analysiert werden.

In dem hier gezeigten Beispiel werden als Verlaufsstrukturmerkmale 202 über die Zeit 203 auftretende Maxima 212 und Minima 222 herangezogen. Dadurch können diejenigen Flussänderungen, welche spezifisch durch das schlagende Herz bedingt sind, besonders zuverlässig erkannt werden. Eine Überlagerung oder ein Vergleich des hier gezeigten Verlaufs 201 mit einem Elektrokardiogramm (EKG) (nicht gezeigt), verdeutlicht den Zusammenhang zwischen den Druckänderungen und den Herzschlägen besonders anschaulich. Das EKG zeigt lediglich die elektrische Aktivität des Herzens, während die vorliegende Erfindung es ermöglicht auch die Pumpaktivität des Herzens zu erfassen. Gut zu erkennen ist hier, dass ein maximaler Betrag des Flusses etwa 0,1 l/s entspricht. Ein solcher, eher geringerer Spitzenfluß ist charakteristisch für die Flussänderungen, welche durch Herzschläge bedingt sind. Die Amplitude der herzschlagbedingten Flusssignale liegt im Bereich 0,5 L/min bis 3,5 l/min beispielsweise bei 0,6 bis 2,3 l/min. Die Amplitude der herzschlagbedingten Drucksignale liegt im Bereich 0,2 cm/H2O bis 3,0 cm/H2O beispielsweise bei 0,4 bis 2,2 cm/H2O.

Zusätzlich oder alternativ kann die Steuereinrichtung 4 für den Verlauf 201 auch eine Mustererkennung durchführen, um Herzschläge zu erkennen. Dazu wird der Verlauf 201 auf das Auftreten eines bestimmten Musters 232 untersucht. In dem hier aufgetragenen Verlauf 201 ist beispielsweise besonders gut ein Muster 232 zu erkennen, welches durch die regelmäßig auftretenden geometrischen Strukturen mit zugehörigen Maxima 212 und Minima 222 sowie den dazwischen liegenden charakteristischen Steigungen geprägt ist. So sind hier in dem Muster besonders gut die spezifischen kleinen, rhythmischen Oszillationen in der Atemgasströmung zu erkennen.

Der zuvor beschriebene Erkennungsmodus kann auch ohne das Beatmungsgerät 1 mit dem Überwachungssystem 10 ausgeführt werden. Dabei umfasst das Überwachungssystem 10 die Überwachungseinrichtung 3 und die Steuereinrichtung 4. Um die Kenngröße 200 der Atemgasströmung zu erfassen, wird die Überwachungseinrichtung 3 mit einer geeigneten Atemschnittstelle an den Patienten gekoppelt.

Das Überwachungssystem 10 kann dann beispielsweise als eigenständiges System bei einer CPR eingesetzt werden, um eine automatisierte Erkennung von Herzschlägen zu ermöglichen. Die Beatmung während der CPR erfolgt dann beispielsweise manuell oder mittels eines separaten und hier nicht näher gezeigten Beatmungsgerätes. Das Überwachungssystem 10 kann auch mit einem bereits vorhandenen Beatmungsgerät 1 gekoppelt oder in dieses integriert werden, um dessen Funktionalität um den Erkennungsmodus zu erweitern.

Das Beatmungsgerät 1 bietet hier einen Betriebsmodus zur Verwendung des Beatmungsgerätes 1 in Kombination mit einer (separat ausgeführten) Herzdruckmassage. Dabei stellt die Beatmungseinrichtung 2 in einem solchen CPR-Betriebsmodus eine für die CPR spezifische Beatmung bereit. So kann die Beatmung vom Beatmungsgerät 1 übernommen werden und der Helfer kann sich auf die HDM konzentrieren. Beispielsweise erfolgen nach 30 Hüben der Herzdruckmassage zwei Beatmungshübe durch die Beatmungseinrichtung 2.

Eine beispielhafte Verwendung des Beatmungsgerätes 1 im Rahmen einer CPR läuft beispielsweise so ab, wie es nachfolgend beschrieben wird. Zunächst wird der Patient bestimmungsgemäß mit dem Beatmungsgerät 1 verbunden. Das Beatmungsgerät 1 wird in den CPR-Betriebsmodus versetzt, damit die Beatmungseinrichtung 2 eine für die CPR besonders geeignete Beatmung bereitstellt. Parallel zur Beatmung oder alternierend erfolgt die Herzdruckmassage durch den Helfer.

Dabei kann das Beatmungsgerät 1 den Helfer bei der HDM assistieren und beispielsweise Rhythmus und Anzahl der HDM akustisch und optisch signalisieren. Es können auch Signale ausgegeben werden, welche für die Qualität der HDM indikativ sind und beispielsweise anzeigen, ob der Druck auf den Brustkorb ausreichend ist. Dazu werden Druckänderungen und Flussänderungen durch die Steuereinrichtung 4 aus den mittels der Überwachungseinrichtung 3 erfassten Signale erkannt und ausgewertet. Da der Druck auf den Brustkorb charakteristische Druckänderungen und Flussänderungen in der Lunge des Patienten bewirkt, bietet das eine zuverlässige Überwachung.

Nach einer bestimmten Anzahl von beispielsweise 30 Hüben fordert das Beatmungsgerät 1 den Helfer auf, die HDM zu unterbrechen. Nun werden mit der Beatmungseinrichtung 2 eine bestimmte Anzahl von beispielsweise zwei Beatmungshüben appliziert. Nach den Beatmungshüben oder auch parallel dazu aktiviert die Steuereinrichtung 4 den Erkennungsmodus zur Erkennung der Herzaktivität. Dazu wird der zeitliche Verlauf 201 der Kenngröße 200 wie oben beschrieben ausgewertet. Beispielsweise werden kardiogene Druckänderungen und/oder Flussänderungen im zeitlichen Verlauf 201 gesucht und, falls diese bestimmte Bedingungen erfüllen, als Herzschläge identifiziert.

Dabei kann vorgesehen sein, dass während der Erfassung der Kenngröße für den zu untersuchenden zeitlichen Verlauf 201 eine Beatmung durch die Beatmungseinrichtung 2 stattfindet. Es kann aber auch vorgesehen sein, dass die Beatmung dann gezielt ausgesetzt wird, beispielsweise um die Erfassung der Kenngröße 200 nicht zu beeinflussen. Das ist dann vorteilhaft, wenn die Messbedingungen schwierig sind bzw. die Signalqualität der Messdaten kritisch ist. Die hier gezeigte Überwachungseinrichtung 3 bzw. Sensoren und die Steuereinheit 4 sind eingerichtet und ausgebildet, die herzschlagbedingten Signale auch während der Beatmung und/oder während der HDM zu erfassen. Die herzschlagbedingten Signale sind dann, bei einer Auftragung der Fluss- oder Drucksignale, als oszillierende Schwankungen der entsprechenden Signale zu erkennen.

Wenn die Steuereinrichtung 4 keine Herzschläge erkennt, wird für den Helfer ein Hinweis ausgegeben, dass die HDM fortgesetzt werden soll. Nun wird die HDM wieder wie zuvor beschrieben ausgeführt und durch das Beatmungsgerät 1 begleitet, bis der nächste Erkennungsmodus erfolgt und so fort. Wenn die Steuereinrichtung 4 Herzschläge erkennt, gibt diese dann einen entsprechenden Hinweis an den Helfer aus.

Ein weiterer beispielhafter Ablauf des Erkennungsmodus wird nun mit Bezug zu der Figur 3 beschrieben. Dabei erfolgt hier zusätzlich zu dem mit Bezug zu der Fig. 2 beschrieben Ablauf noch eine Plausibilitätsüberprüfung, um das das Ergebnis der Herschlagerkennung zu verifizieren.

Dazu wertet die Steuereinrichtung 4 einen über die Zeit 303 registrierten Verlauf 301 einer weiteren Kenngröße 300 aus. Die weitere Kenngröße 300 ist hier ein Kohlendioxidpartialdruck des Atemgases, welchen die Überwachungseinrichtung 3 mit einem eigenen oder einem externen Sensormittel erfasst hat.

Es hat sich gezeigt, dass zu dem Zeitpunkt 302, an dem die Kreislaufaktivität wieder einsetzt, auch ein signifikanter Anstieg des Kohlendioxidpartialdrucks im Atemgases auftritt. Daher überprüft die Steuereinrichtung 4 hier den Verlauf 301 der weiteren Kenngröße 300 auf einen solchen Anstieg des Kohlendioxidpartialdrucks. Wenn der Anstieg vorliegt, kann z. B. ein Hinweis ausgegeben werden. Wenn kein Anstieg erfolgt, kann z. B. ein Hinweis zur Warnung ausgegeben werden oder der Erkennungsmodus fortgesetzt oder das Ergebnis verworfen werden.

Alternativ oder zusätzlich kann auch eine pulsoxymetrisch erfasste Plethysmogramm-Welle und/oder eine pulsoxymetrisch gemessene Sauerstoffkonzentration des Blutes als weitere Kenngröße 300 herangezogen werden. Dann ist die Überwachungseinrichtung 3 z. B. mit einem Pulsoxymeter wirkverbunden. So kann z. B. anhand einer signifikanten Veränderung der Sauerstoffkonzentration festgestellt werden, ob die Kreislaufaktivität wieder eingesetzt hat.

### Bezugszeichenliste:

- 1: Beatmungsgerät
- 2: Beatmungseinrichtung
- 3: Überwachungseinrichtung
- 4: Steuereinrichtung
- 5: Sensormittel
- 6: Ausgabeeinrichtung
- 7: Bedieneinrichtung
- 10: Überwachungssystem
- 12: Lüftereinrichtung
- 22: Atemmaske
- 32: Schlaucheinrichtung
- 200: Kenngröße
- 201: Verlauf
- 202: Verlaufsstrukturmerkmal
- 203: Zeit
- 212: Maximum
- 222: Minimum
- 232: Muster
- 300: Kenngröße
- 301: Verlauf
- 302: Zeitpunkt
- 303: Zeit

## Patentansprüche

1. Beatmungsgerät (1) mit wenigstens einer Beatmungseinrichtung (2) zur Erzeugung einer Atemgasströmung für eine Beatmung und mit wenigstens einer Überwachungseinrichtung (3) zur Überwachung wenigstens einer charakteristischen Kenngröße (200) der Atemgasströmung, wobei die wenigstens eine Kenngröße (200) ein Maß für einen Fluss des Atemgases ist,
und mit wenigstens einer Steuereinrichtung (4), welche dazu geeignet und ausgebildet ist, wenigstens einen Erkennungsmodus für eine Herzaktivität auszuführen und dazu einen zeitlichen Verlauf (201) der Kenngröße (200) der Atemgasströmung zu registrieren und den zeitlichen Verlauf (201) der Kenngröße (200) auf wenigstens ein Verlaufsstrukturmerkmal (202) zu untersuchen und Herzschläge wenigstens dadurch zu erkennen, dass das Verlaufsstrukturmerkmal (202) wenigstens eine hinterlegte Bedingung für ein herzschlagbedingtes Verlaufsstrukturmerkmal (202) wenigstens teilweise erfüllt, **dadurch gekennzeichnet,**
**dass** das wenigstens eine Verlaufsstrukturmerkmal (202) ein Auftreten von Maxima (212) und/oder Minima (222) im zeitlichen Verlauf (201) des Flusses beschreibt und dass als Bedingung wenigstens eine maximale Grenze für einen Betrag des Flusses an einem Maximum (212) und an einem Minimum (222) vorgesehen ist
und **dass** als Bedingung wenigstens eine Obergrenze zwischen 0,01 Liter pro Sekunde und 0,3 Liter pro Sekunde für einen Betrag des Flusses vorgesehen ist.

2. Beatmungsgerät (1) nach dem vorhergehenden Anspruch, wobei das wenigstens eine Verlaufsstrukturmerkmal (202) eine zeitliche Änderung der Kenngröße (200) beschreibt und wobei als Bedingung wenigstens ein Maß für eine Ähnlichkeit zu einer durch Herzschläge verursachten zeitlichen Änderung der Kenngröße (200) vorgesehen ist.

3. Beatmungsgerät (1) nach einem der vorhergehenden Ansprüche, wobei das wenigstens eine Verlaufsstrukturmerkmal (202) eine zeitliche Flussänderung beschreibt und wobei die Bedingung wenigstens ist, dass die zeitliche Flussänderung eine definierte Ähnlichkeit zu einer durch Herzschläge verursachten zeitlichen Flussänderung aufweist.

4. Beatmungsgerät (1) nach einem der vorhergehenden Ansprüche, wobei als Bedingung wenigstens vorgegeben ist, wie häufig und/oder regelmäßig das wenigstens eine Verlaufsstrukturmerkmal (202) im zeitlichen Verlauf (201) der Kenngröße (200) auftritt

5. Beatmungsgerät (1) nach einem der vorhergehenden Ansprüche, wobei die Obergrenze für den Betrag des Flusses des Atemgases zwischen 0,02 Liter pro Sekunde und 0,15 Liter pro Sekunde liegt.

6. Beatmungsgerät (1) nach einem der vorhergehenden Ansprüche, wobei als Bedingung wenigstens vorgesehen ist, dass zeitliche Änderungen der Kenngröße (200) und vorzugsweise ein Auftreten von Maxima (212) und/oder Minima (222) im zeitlichen Verlauf (201) der Kenngröße (200) mit einer Frequenz von wenigstens 10 pro Minute und vorzugsweise 20 pro Minute und/oder im Bereich 30 bis 200 pro Minute erfolgen.

7. Beatmungsgerät (1) nach einem der vorhergehenden Ansprüche, wobei die Steuereinrichtung (4) dazu geeignet und ausgebildet ist, eine Häufigkeit des wenigstens einen Verlaufsstrukturmerkmals (202) im zeitlichen Verlauf (201) der Kenngröße (200) zu bestimmen und aus der Häufigkeit eine Anzahl von Herzschlägen und/oder eine Herzfrequenz zu bestimmen.

8. Beatmungsgerät (1) nach einem der vorhergehenden Ansprüche, wobei die Steuereinrichtung (4) dazu geeignet und ausgebildet ist, die Erkennung der Herzschläge unter Berücksichtigung davon vorzunehmen, ob das wenigstens eine Verlaufsstrukturmerkmal (202) mit einer Mindestqualität und/oder Mindestanzahl im registrierten zeitlichen Verlauf (201) der Kenngröße (200) aufgetreten ist und/oder ob der zeitliche Verlauf (201) der Kenngröße (200) der Atemgasströmung während einer Mindestdauer registriert wurde und/oder ob der zeitliche Verlauf (201) der Kenngröße (200) der Atemgasströmung mit einer definierten Mindestsignalqualität registriert wurde und wobei die Mindestdauer insbesondere wenigstens fünf Sekunden beträgt.

9. Beatmungsgerät (1) nach einem der vorhergehenden Ansprüche, wobei mittels der Überwachungseinrichtung (3) ein Kohlendioxidgehalt des Bluts und/oder des Atemgases und/oder ein Blutdruck und/oder eine Sauerstoffkonzentration des Blutes als wenigstens eine weitere Kenngröße (200) erfassbar sind und wobei die Steuereinrichtung (4) dazu geeignet und ausgebildet ist, einen zeitlichen Verlauf (201) der wenigstens einen weiteren Kenngröße (200) zu registrieren und für die Erkennung der Herzschläge wenigstens teilweise zu berücksichtigen und insbesondere wobei die weitere Kenngröße eine pulsoxymetrisch erfasste Plethysmogramm-Welle und/oder eine pulsoxymetrisch gemessene Sauerstoffkonzentration ist.

10. Beatmungsgerät (1) nach einem der vorhergehenden Ansprüche, wobei die Steuereinrichtung (4) dazu geeignet und ausgebildet ist, zur Überwachung einer Herzdruckmassage einen zeitlichen Verlauf (201) eines Flusses des Atemgases wenigstens während der Herzdruckmassage zu registrieren und eine Wirkung der Herzdruckmassage in Abhängigkeit davon zu bewerten, dass der zeitliche Verlauf (201) eine definierte zeitliche Flussänderung aufweist.

11. Beatmungsgerät (1) nach einem der vorhergehenden Ansprüche, wobei die Steuereinrichtung (4) dazu geeignet und ausgebildet ist, für eine Erkennung der Herzschläge wenigstens einen Hinweis auszugeben, dass eine Herzdruckmassage ausgesetzt und/oder wieder begonnen werden soll und/oder wobei die Steuereinrichtung (4) dazu geeignet und ausgebildet ist, wenigstens einen akustischen und/oder optischen und/oder haptischen Unterstützungshinweis für eine Herzdruckmassage und vorzugsweise für deren Rhythmus vorzugeben.

12. Beatmungsgerät (1) nach einem der vorhergehenden Ansprüche, wobei die Beatmungseinrichtung (2) mittels der Steuereinrichtung (4) in wenigstens einem Betriebsmodus zur Verwendung des Betatmungsgeräts in Kombination mit einer Herzdruckmassage betreibbar ist und wobei die Beatmungseinrichtung (2) in dem Betriebsmodus wenigstens eine für eine kardiopulmonale Reanimation (englisch Cardiopulmonary Resuscitation, CPR) spezifische Beatmung bereitstellt (sog. CPR-Betriebsmodus).

13. Beatmungsgerät (1) nach dem vorhergehenden Anspruch, wobei als Bedingung wenigstens vorgesehen ist, dass eine zeitliche Änderung der Kenngröße, vorzugsweise Flussänderung, wenigstens eine Amplitude aufweist, deren Größe maximal die Hälfte, vorzugsweise maximal ein Viertel, der Größe wenigstens einer Amplitude beträgt, welche die zeitliche Änderung der Kenngröße während der Durchführung einer HDM aufweist.

14. Beatmungsgerät (1) nach einem der beiden vorhergehenden Ansprüche, wobei als Bedingung wenigstens vorgesehen ist, dass eine zeitliche Änderung der Kenngröße, vorzugsweise Flussänderung, wenigstens eine Amplitude aufweist, deren Größe maximal die Hälfte, vorzugsweise maximal ein Drittel, der Größe wenigstens einer Amplitude beträgt, welche für den Fluss des Atemgases für die Beatmung während des CPR-Betriebsmodus vorgesehen bzw. eingestellt ist.

## Claims

1. A ventilator (1) comprising at least one ventilation apparatus (2) for generating a respiratory gas flow for ventilation, and comprising at least one monitoring apparatus (3) for monitoring at least one characteristic parameter (200) of the respiratory gas flow, wherein the at least one characteristic parameter (200) is a measure of a flow of the respiratory gas, and comprising at least one control apparatus (4) which is suitable and designed to carry out at least one detection mode for cardiac activity and, for this purpose, to register a time profile (201) of the characteristic parameter (200) of the respiratory gas flow and to examine the time profile (201) of the characteristic parameter (200) for at least one profile-structure feature (202), and to detect heartbeats at least in that the profile-structure feature (202) at least partially satisfies at least one stored condition for a heartbeat-related profile-structure feature (202), **characterized in that** the at least one profile-structure feature (202) describes an occurrence of maxima (212) and/or minima (222) in the time profile (201) of the flow, and **in that** at least one maximum limit for an amount of the flow at a maximum (212) and at a minimum (222) is provided as a condition, and **in that** at least one upper limit between 0.01 liters per second and 0.3 liters per second for an amount of the flow is provided as a condition.

2. The ventilator (1) according to the preceding claim,
wherein the at least one profile-structure feature (202) describes a temporal change in the characteristic parameter (200), and wherein at least one measure of a similarity to a temporal change in the characteristic parameter (200) caused by heartbeats is provided as a condition.

3. The ventilator (1) according to one of the preceding claims, wherein the at least one profile-structure feature (202) describes a temporal flow change, and wherein the condition is at least that the temporal flow change has a defined similarity to a temporal flow change caused by heartbeats.

4. The ventilator (1) according to one of the preceding claims, wherein it is at least provided as a condition how often and/or regularly the at least one profile-structure feature (202) occurs in the time profile (201) of the characteristic parameter (200).

5. The ventilator (1) according to one of the preceding claims, wherein the upper limit for the amount of the flow of the respiratory gas is between 0.02 liters per second and 0.15 liters per second.

6. The ventilator (1) according to any of the preceding claims, wherein it is at least provided as a condition that temporal changes in the characteristic parameter (200), and preferably an occurrence of maxima (212) and/or minima (222) in the time profile (201) of the characteristic parameter (200), occur at a frequency of at least 10 per minute and preferably 20 per minute and/or in the range from 30 to 200 per minute.

7. The ventilator (1) according to one of the preceding claims, wherein the control apparatus (4) is suitable and designed to determine a frequency of the at least one profile-structure feature (202) in the time profile (201) of the characteristic parameter (200) and to determine, from the frequency, a number of heartbeats and/or a heart rate.

8. The ventilator (1) according to one of the preceding claims, wherein the control apparatus (4) is suitable and designed to perform the detection of the heartbeats taking into account whether the at least one profile-structure feature (202) has occurred with a minimum quality and/or minimum number in the registered time profile (201) of the characteristic parameter (200) and/or whether the time profile (201) of the characteristic parameter (200) of the respiratory gas flow has been registered for a minimum duration and/or whether the time profile (201) of the characteristic parameter (200) of the respiratory gas flow has been registered with a defined minimum signal quality, and wherein the minimum duration is in particular at least five seconds.

9. The ventilator (1) according to one of the preceding claims, wherein, by means of the monitoring apparatus (3), a carbon dioxide content of the blood and/or of the respiratory gas and/or a blood pressure and/or an oxygen concentration of the blood can be recorded as at least one further characteristic parameter (200) and wherein the control apparatus (4) is suitable and designed to register a time profile (201) of the at least one further characteristic parameter (200) and to at least partially take it into account for the detection of the heartbeats, and in particular wherein the further characteristic parameter is a plethysmogram wave recorded by pulse oximetry and/or an oxygen concentration measured by pulse oximetry.

10. The ventilator (1) according to one of the preceding claims, wherein the control apparatus (4) is suitable and designed to register, in order to monitor chest compressions, a time profile (201) of a flow of the respiratory gas at least during the chest compressions, and to evaluate an effect of the chest compressions depending on the fact that the time profile (201) has a defined temporal flow change.

11. The ventilator (1) according to one of the preceding claims, wherein the control apparatus (4) is suitable and designed to output, for a detection of the heartbeats, at least an indication that chest compressions are to be paused and/or resumed, and/or wherein the control apparatus (4) is suitable and designed to provide at least one acoustic and/or visual and/or haptic support indication for chest compressions and preferably for a rhythm thereof.

12. The ventilator (1) according to one of the preceding claims, wherein the ventilation apparatus (2) is operable by means of the control apparatus (4) in at least one operating mode for use of the ventilator in combination with chest compressions, and wherein the ventilation apparatus (2) in the operating mode provides at least specific ventilation for cardiopulmonary resuscitation (CPR) (so-called CPR operating mode).

13. The ventilator (1) according to the preceding claim,
wherein it is at least provided as a condition that a temporal change in the characteristic parameter, preferably a flow change, has at least one amplitude, the magnitude of which is at most half, preferably at most a quarter, of the magnitude of at least one amplitude which the temporal change in the characteristic parameter has during the performance of chest compressions.

14. The ventilator (1) according to either of the two preceding claims, wherein it is at least provided as a condition that a temporal change in the characteristic parameter, preferably a flow change, has at least one amplitude, the magnitude of which is at most half, preferably at most one third, of the magnitude of at least one amplitude which is provided or, respectively, set for the flow of the respiratory gas for the ventilation during the CPR operating mode.

## Revendications

1. Appareil respiratoire (1) comprenant au moins une unité de ventilation (2) destinée à générer un flux de gaz respiratoire pour une ventilation et comprenant au moins une unité de surveillance (3) destinée à surveiller au moins une grandeur caractéristique (200) du débit de gaz respiratoire, dans lequel l'au moins une grandeur caractéristique (200) constitue une mesure d'un débit du gaz respiratoire, et comprenant au moins une unité de commande (4) adaptée et configurée pour exécuter au moins un mode de détection d'une activité cardiaque et, à cet effet, pour enregistrer une évolution temporelle (201) de la grandeur caractéristique (200) du débit de gaz respiratoire et pour analyser l'évolution temporelle (201) de la grandeur caractéristique (200) en vue d'y rechercher au moins une particularité structurelle d'évolution (202), et pour détecter des battements cardiaques au moins en ce que la particularité structurelle d'évolution (202) satisfait au moins partiellement une condition prédéfinie pour une particularité structurelle d'évolution (202) liée à un battement cardiaque, **caractérisé en ce que** l'au moins une particularité structurelle d'évolution (202) décrit une occurrence de maxima (212) et/ou de minima (222) dans l'évolution temporelle (201) du débit, et **en ce qu'**est prévue comme condition au moins une limite maximale pour une valeur absolue du débit à un maximum (212) et à un minimum (222), et **en ce qu'**est prévue comme condition au moins une limite supérieure comprise entre 0,01 litre par seconde et 0,3 litre par seconde pour un valeur absolue du débit.

2. Appareil respiratoire (1) selon la revendication précédente, dans lequel l'au moins une particularité structurelle d'évolution (202) décrit une variation temporelle de la grandeur caractéristique (200), et dans lequel au moins une mesure d'une similarité avec une variation temporelle de la grandeur caractéristique (200) provoquée par des battements cardiaques est prévue comme condition.

3. Appareil respiratoire (1) selon l'une au moins des revendications précédentes, dans lequel l'au moins une particularité structurelle d'évolution (202) décrit une variation temporelle du débit, et dans lequel la condition consiste au moins dans le fait que la variation temporelle du débit présente une similarité définie avec une variation temporelle du débit provoquée par des battements cardiaques.

4. Appareil respiratoire (1) selon l'une au moins des revendications précédentes, dans lequel la condition spécifie au moins à quelle fréquence et/ou avec quelle régularité l'au moins une particularité structurelle d'évolution (202) apparaît dans l'évolution temporelle (201) de la grandeur caractéristique (200).

5. Appareil respiratoire (1) selon l'une au moins des revendications précédentes, dans lequel la limite supérieure relative à la valeur absolue du débit du gaz respiratoire se situe entre 0,02 litre par seconde et 0,15 litre par seconde.

6. Appareil respiratoire (1) selon l'une au moins des revendications précédentes, dans lequel il est prévu comme condition au moins que des variations temporelles de la grandeur caractéristique (200) et, de préférence, une occurrence de maxima (212) et/ou de minima (222) dans l'évolution temporelle (201) de la grandeur caractéristique (200), se produisent à une fréquence d'au moins 10 par minute et, de préférence, 20 par minute et/ou dans une plage allant de 30 à 200 par minute.

7. Appareil respiratoire (1) selon l'une au moins des revendications précédentes, dans lequel l'unité de commande (4) est adaptée et configurée pour déterminer un taux d'occurrence de l'au moins une particularité structurelle d'évolution (202) dans l'évolution temporelle (201) de la grandeur caractéristique (200) et pour déterminer, à partir du taux d'occurrence, un nombre de battements cardiaques et/ou une fréquence cardiaque.

8. Appareil respiratoire (1) selon l'une au moins des revendications précédentes, dans lequel l'unité de commande (4) est adaptée et configurée pour effectuer la détection des battements cardiaques en tenant compte du fait que l'au moins une particularité structurelle d'évolution (202) est apparue avec une qualité minimale et/ou un nombre minimal dans l'évolution temporelle (201) enregistrée de la grandeur caractéristique (200) et/ou du fait que l'évolution temporelle (201) de la grandeur caractéristique (200) du flux de gaz respiratoire a été enregistrée pendant une durée minimale et/ou du fait que l'évolution temporelle (201) de la grandeur caractéristique (200) du flux de gaz respiratoire a été enregistrée avec une qualité de signal minimale définie, la durée minimale étant en particulier d'au moins cinq secondes.

9. Appareil respiratoire (1) selon l'une au moins des revendications précédentes, dans lequel l'unité de surveillance (3) permet de détecter une teneur en dioxyde de carbone du sang et/ou du gaz respiratoire et/ou une pression artérielle et/ou une concentration en oxygène du sang en tant qu'au moins une autre grandeur caractéristique (200), et l'unité de commande (4) est adaptée et configurée pour enregistrer une évolution temporelle (201) de l'au moins une autre grandeur caractéristique (200) et pour la prendre en compte au moins partiellement pour la détection des battements cardiaques, dans lequel ladite autre grandeur caractéristique est en particulier une onde de pléthysmogramme détectée par oxymétrie de pouls et/ou une concentration en oxygène mesurée par oxymétrie de pouls.

10. Appareil respiratoire (1) selon l'une au moins des revendications précédentes, dans lequel l'unité de commande (4) est adaptée et configurée pour enregistrer, pour la surveillance d'un massage cardiaque externe, une évolution temporelle (201) d'un débit du gaz respiratoire au moins durant le massage cardiaque externe, et pour évaluer un effet du massage cardiaque externe en fonction du fait que l'évolution temporelle (201) présente une variation temporelle définie du débit.

11. Appareil respiratoire (1) selon l'une au moins des revendications précédentes, dans lequel l'unité de commande (4) est adaptée et configurée pour délivrer, pour une détection des battements cardiaques, au moins une indication selon laquelle un massage cardiaque externe doit être suspendu et/ou repris, et/ou dans lequel l'unité de commande (4) est adaptée et configurée pour fournir au moins une indication d'assistance acoustique et/ou optique et/ou haptique pour un massage cardiaque externe et, de préférence, pour le rythme de celui-ci.

12. Appareil respiratoire (1) selon l'une au moins des revendications précédentes, dans lequel l'unité de ventilation (2) peut être actionnée au moyen de l'unité de commande (4) dans au moins un mode de fonctionnement destiné à l'utilisation de l'appareil respiratoire en combinaison avec un massage cardiaque externe, et dans lequel l'unité de ventilation (2), dans ledit mode de fonctionnement, fournit au moins une ventilation spécifique à une réanimation cardiopulmonaire (Cardiopulmonary Resuscitation, CPR) (dit mode de fonctionnement CPR).

13. Appareil respiratoire (1) selon la revendication précédente, dans lequel il est prévu comme condition au moins qu'une variation temporelle de la grandeur caractéristique, de préférence une variation du débit, présente au moins une amplitude dont la valeur est au maximum la moitié, de préférence au maximum un quart, de la valeur d'au moins une amplitude que présente la variation temporelle de la grandeur caractéristique lors de l'exécution d'un massage cardiaque externe.

14. Appareil respiratoire (1) selon l'une au moins des deux revendications précédentes, dans lequel il est prévu comme condition au moins qu'une variation temporelle de la grandeur caractéristique, de préférence une variation du débit, présente au moins une amplitude dont la valeur est au maximum la moitié, de préférence au maximum un tiers, de la valeur d'au moins une amplitude prévue ou réglée pour le débit du gaz respiratoire pour la ventilation durant le mode de fonctionnement CPR.
